# EUROPEAN PATENT SPECIFICATION

(11) **EP 1 680 020 B1**
(45) Date of publication and mention of the grant of the patent: **17.06.2015**
(21) Application number: 03786528.4
(22) Date of filing: 14.10.2003
(51) Int. Cl.: A61B 5/053, A61B 5/11, A61B 5/00, A61B 5/08, A61B 5/02, A61B 5/113, A61N 1/365

(54) **DETECTION OF CONGESTION FROM MONITORING PATIENT RESPONSE TO A RECUMBENT POSITION**
DETEKTIERUNG EINER KONGESTION DURCH ÜBERWACHUNG DER PATIENTENREAKTION IN DER LIEGENDEN POSITION
DETECTION DE CONGESTION EFFECTUEE PAR SURVEILLANCE D'UNE REACTION D'UN PATIENT A UNE POSITION ALLONGEE

(43) Date of publication of application: 19.07.2006
(60) Divisional application: 11177688.6
(73) Proprietor: CARDIAC PACEMAKERS, INC., St. Paul, Minnesota 55112-5798 (US)
(72) Inventor: HATLESTAD, John, Maplewood, MN 55117 (US); ZHU, Qingsheng, Little Canada, MN 55117 (US); STAHMANN, Jeffrey, E., Ramsey, MN 55303 (US)
(74) Representative: Golkowsky, Stefan
(86) International application number: PCT/US2003/032553
(87) International publication number: WO 2005/046472

(56) References cited:
- EP-A- 0 985 429
- EP-A- 1 151 719
- WO-A-01/19426
- WO-A1-02/40096
- US-A- 6 047 203
- US-A1- 2002 170 193
- US-A1- 2003 055 461
- US-A1- 2004 073 128

## Description

### TECHNICAL FIELD

The present invention is related to the detection of congestion of a patient. More particularly, the present invention is related to the detection of congestion through monitoring a patient's response to a recumbent position of the patient's body.

### BACKGROUND

A patient suffers from congestion when filling pressure of the heart, which results from the return of blood from the body to the heart, is relatively high due to the heart's inability to effectively pump blood back out to the body. Thus, congestion is indicative of heart failure and is one factor that should be closely monitored for a heart failure patient. Congestion may be manifested in several ways in the heart failure patient's body.

One condition highly correlated with congestion is respiratory distress upon lying down. Congestion often results in the lungs becoming partially filled with fluid once the patient lies down, and the lungs maintain the partially filled state until the patient becomes upright. This filling causes the patient to have difficulty breathing while in the recumbent position, and the respiratory distress that may result from the lungs remaining filled with fluid is a good indicator of congestion.

Attempts have been made to assess congestion from observation of a patient's posture relative to his average trans-thoracic impedance, which decreases as the lungs fill with fluid. However, the average trans-thoracic impedance masks any change that might occur in the patient's respiratory patterns. Thus, average trans-thoracic impedance measurements fail to indicate the patient's level of respiratory distress that results from the recumbent position.

EP 0 985 429 B1, US 2003/0055461 A1, and WO 02/40096 all disclose systems for obtaining information related to congestion of a patient comprising a reclination sensor, detection circuitry, and a respiration sensor that produces a respiration signal indicative of respiration patterns of the patient. All inventions of said documents rely on impedance data to determine the status of congestive heart failure.

### SUMMARY

The present invention address the issues discussed above and others by providing a system according to claim 1 that obtain information that allows detection of congestion by assessing the patient's respiratory distress. The respiratory distress is assessed based on a comparison of respiration pattern

The information related to congestion is obtained by first detecting whether the patient is in a recumbent position. When the patient is not in the recumbent position, the respiration of the patient is monitored to detect a first respiration pattern. When the patient is in the recumbent position, the respiration of the patient is monitored to detect a second respiration pattern. Differences in the two breathing patterns may indicate respiratory distress due to the recumbent position, which is a sign of congestion.

### DESCRIPTION OF THE DRAWINGS

FIG. 1 illustrates one or more devices coupled to a patient to obtain information allowing the detection of congestion by monitoring the patient's response to a recumbent position.
FIG. 2 shows a diagram of major components of one embodiment of a system for obtaining information allowing the detection of congestion by comparing an upright respiration pattern of the patient to a recumbent respiration pattern.
FIG. 3 shows an example of operational flow for the system of FIG. 2 for obtaining information related to congestion and for detecting congestion from the information.
FIG. 4 depicts first and second respiration patterns whose difference indicates congestion.
FIG. 5 shows a diagram of major components of an examplary system for obtaining information allowing the detection of congestion by determining the amount of time a patient remains in a recumbent position relative to a threshold indicative of congestion and/or by determining the recumbent angle that the patient obtains relative to a threshold indicative of a normal recumbent angle.
FIG. 6 shows an example of operational flow for the system of FIG. 5 to determine the amount of time that the patient remains in the recumbent position.
FIG. 7 shows an example of operational flow for the system of FIG. 5 to determine a recumbent angle that the patient obtains.

### DETAILED DESCRIPTION

Embodiments of the present invention attempt to obtain information related to a congestive condition and/or to diagnose a congestive condition of a patient from the information by monitoring the patient's response to a recumbent position of the patient's body. For patients suffering from congestion, respiratory distress will likely occur at some point after the patient's body achieves a recumbent state. The respiratory distress is measured by comparison of respiratory patterns detected during the recumbent state and during a non-recumbent state.

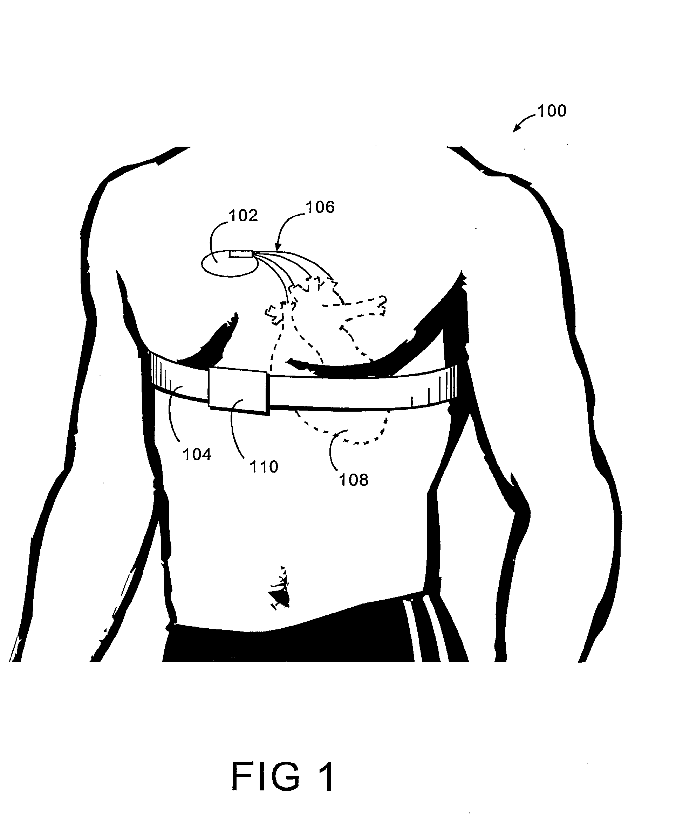

FIG. 1 shows a patient's body 100 having one or more devices coupled to the body 100 to detect and/or infer respiratory distress occurring once the body 100 becomes recumbent. For example, an implantable medical device (IMD) 102 such as a pacemaker or device dedicated to congestion detection may be implanted in the body 100 and may be configured to monitor the patient's behavior in relation to a recumbent state, such as by detecting a change in respiratory patterns, finding the amount of time the patient remains in the recumbent state, or finding the recumbent angle the patient is able to obtain. Alternatively, an external device such as a respiratory effort band 104 may be coupled to the torso of the body 100 to monitor the patient's respiration in relation to the recumbent state by measuring the chest movement caused by the respiratory patterns.

In situations where an IMD 102 is utilized, the IMD 102 may make respiratory measurements as part of its routine for providing therapy to the patient, such as a pacemaker measuring respiratory rate to control pulse rate of the patient's heart 108. These respiratory measurements can also be utilized, either by the IMD 102 or another device to detect respiratory patterns of the patient. One manner of detecting the respiratory patterns is through monitoring trans-thoracic impedance variation through one or more leads 106 during each individual inhale and exhale cycle. The IMD 102 or other device coupled to the body 100 is provided with a reclination sensor, such as a tilt switch, to allow a distinction between the body 100 being in a recumbent or non-recumbent state. Furthermore, the reclination sensor may be an accelerometer to allow the recumbent angle of the patient to be measured.

In situations where an external device 104 is utilized, the external device 104 can similarly make respiratory measurements and sense the recumbent state of the body 100. For example, a respiratory effort band measures the expansion and contraction of the torso of the body 100 resulting from respiratory cycles to produce signals that represent the respiratory patterns of the patient. The external device 104 or other device also detects the reclination using a tilt switch or accelerometer to distinguish between the recumbent and non-recumbent states. The external device 104 includes a module 110 that contains electrical circuitry discussed below for obtaining information related to congestion and may also detect congestion from the obtained information.

For example, the IMD 102 or external device 104 may measure the reclination of the patient and then employ a clock to track the amount of time that the body 100 is in a recumbent state. The amount of time may then be compared to a predetermined threshold to infer whether the patient has respiratory distress while in a recumbent state. The inference may be made because it may be presumed that the patient becomes non-recumbent once respiratory distress begins to occur to relieve the discomfort.

As another example, the IMD 102 or external device 104 may measure the recumbent angle obtained by the patient for a period of time, such as the maximum recumbent angle or an average recumbent angle that the patient achieves while sleeping. The recumbent angle may then be compared to a predetermined threshold, such as a typical recumbent angle for a healthy patient, to infer whether the patient suffers respiratory distress at recumbent angles greater than the recumbent angle actually measured for the patient. The inference may be made because it may be presumed that the patient achieves the greatest recumbent angle possible while resting that does not result in discomfort due to respiratory distress.

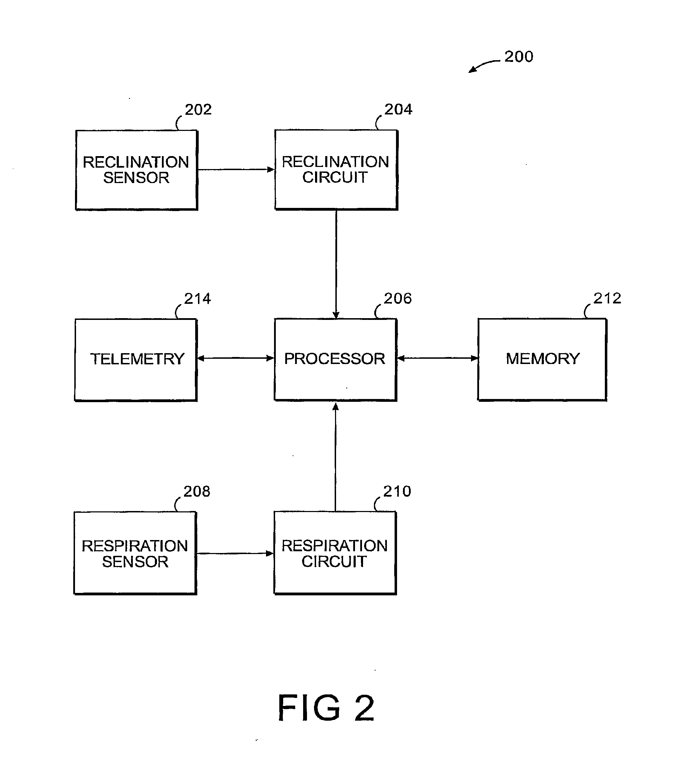

FIG. 2 shows the major components of an embodiment of a system 200 that detects respiratory distress caused by a recumbent position of the body 100. As discussed above, any or all of the components of system 200 may be internal or external to the body 100. For example, any or all of the components may be contained within the IMD 102 and/or module 110 of external device 104.

The system 200 includes a reclination sensor 202 the produces an electrical signal that is representative of the orientation of the reclination sensor 202 relative to gravity. One example of a reclination sensor 202 is a tilt switch. Another example is a DC accelerometer. In either case, a signal is produced by the reclination sensor 202 in response to the reclination sensor 202 having a particular orientation relative to gravity. Therefore, the reclination sensor 202 is coupled to the body 100 with a known orientation so that the reclination sensor 202 produces a detectable signal in response to the body 100 having a recumbent or non-recumbent state. The reclination sensor 202 may be multi-axis responsive so that regardless of the orientation of the body 100 while in a recumbent position (i.e., lying on back versus lying on side), a detectable signal is produced.

A reclination circuit 204 is electrically connected to the reclination sensor 202 to convert the reclination sensor signal into digital data that may be processed. For a tilt switch, the conversion may be simply recognizing the switch as open or closed, where one state indicates a recumbent patient and the other state indicates a non-recumbent patient. For an accelerometer, the reclination circuit 204 may amplify and/or filter the accelerometer signal prior to conversion to digital data to maintain a sufficient granularity for more precisely determining the degree of reclination of the body 100. The reclination circuit 204 then provides the reclination data to a processing device 206 where the degree of reclination of the body 100 can be factored into the determination of congestion.

A respiration sensor 208 detects the respiration patterns of the body 100 and produces a signal representative of the respiration patterns. The respiration sensor 208 may be of various forms such as a trans-thoracic impedance sensor of an IMD 202. Other forms are possible as well, including the respiratory effort band 104 whose impedance varies as the strap 104 expands and contracts in relation to the chest of the body 100 expanding and contracting.

The signal representative of respiration is provided to a respiration circuit 210 that produces digital data from the respiration signal that can be further processed and factored into the detection of congestion. The respiration circuit 210 may amplify and/or filter the respiration signal received from the respiration sensor 208. The respiration circuit 210 then provides the respiration data that identifies the respiration pattern to the processing device 206.

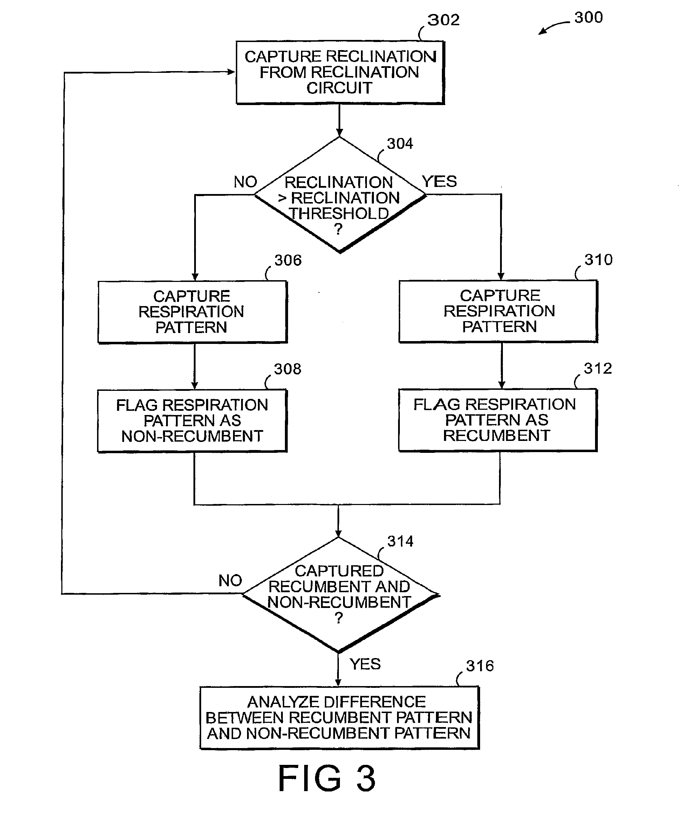

The processing device 206 may be a general-purpose programmable processor, hardwired digital logic, or other suitable processing device for applying logical operations to the reclination and respiration data. One example of the logical operations of the processing device 206 are described below with reference to FIG. 3. The processor 206 utilizes memory 212 for programming and/or storage of received and processed data while implementing the logical operations of FIG. 3.

In the embodiment shown in FIG. 2, the system 200 includes telemetry 214 that allows the processor 206 to communicate information to an external device (not shown) used by the physician monitoring the patient. For example, the IMD 102 employs telemetry 214 to communicate bi-directionally and wirelessly with a device programmer. An external device 104 may also employ telemetry 214 to communicate either wirelessly or through a wired connection to a device such as a programmer. The programmer may also communicate back to the device 200, such as to update instructions stored in memory 212 that are employed by the processor 206 to bring about the logical operations of FIG. 3.

The illustrative logical operations 300 of FIG. 3 are performed by the processor 206 to detect whether the patient is suffering from congestion by finding whether the respiratory distress is experienced when lying down. The logical operations begin at data operation 302 where the processor 206 receives reclination data from the reclination circuit 204. At query operation 304, the processor 206 detects whether the reclination of the torso of body 100 is greater than a reclination threshold stored in memory 212. The reclination threshold may be chosen based on the desired amount of reclination necessary to be considered recumbent. This value may be patient specific as some patients may choose to sleep or recline to different degrees. For example, reclination of at least 65 degrees away from vertical may be considered a recumbent position for one patient while reclination of at least 85 degrees away from vertical is considered a recumbent position for another.

If the reclination is not greater than the reclination threshold as determined by query operation 304, then the processor 206 accepts data from the respiration circuit 210 at data operation 306 and flags the received data as non-recumbent data at flag operation 308. If the reclination is greater than the reclination threshold as determined at query operation 304, then the processor 206 accepts data from the respiration circuit 210 at data operation 310 and flags that data as recumbent data at flag operation 312.

Query operation 314 detects whether the processor 206 has received and flagged a sufficient quantity of both recumbent data and non-recumbent data. If not, then operational flow returns to data operation 302 where the processor 206 continues to receive reclination data. If query operation 314 finds that a sufficient quantity of both recumbent and non-recumbent data have been received and flagged, then operational flow proceeds to analysis operation 316.

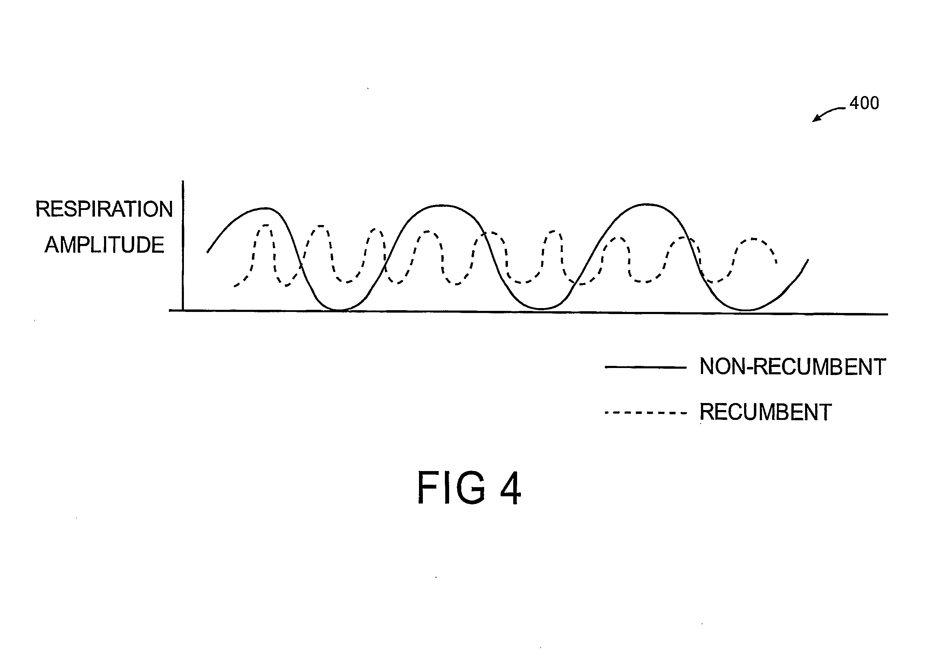

At analysis operation 316, the processor 206 compares the two respiration patterns represented by the respiration data flagged as recumbent data and non-recumbent data. FIG. 4 shows two illustrative hypothetical respiration patterns. The non-recumbent respiration pattern shows that the patient is taking relatively slow and deep breaths as can be seen by the relatively low frequency and high amplitude of the pattern. However, the recumbent respiration pattern shows that the patient is taking relatively rapid and shallow breaths as indicated by the relatively high frequency and low amplitude of the pattern. The rapid and shallow breathing of the recumbent respiration pattern indicates a patient suffering from orthopnea, or paroxysmal nocturnal dyspnea ("PND") that eventually occurs upon lying down.

The presence of orthopnea or PND such as shown in FIG. 4 is known to be a sign of congestion. However, other recumbent respiration pattern changes resulting from lying down may also be indicative of congestion. Therefore, at analysis operation 316 the processor 206 may perform various comparisons in addition to or as an alternative to looking for both rapid and shallow breaths. For example, the processor 206 may search for only rapid recumbent respiration relative to upright respiration. Similarly, the processor 206 may search for only shallow, or low tidal volume, recumbent respiration relative to upright respiration. As another example, the processor 206 may search for a difference in the combination of respiratory rate to tidal volume between the recumbent and non-recumbent respiration patterns. Such a combination may be a ratio of respiratory rate to tidal volume. Additionally, the processor 206 may search for a difference in inspiratory times and expiratory times, inspiratory time of a recumbent pattern versus inspiratory for a non-recumbent pattern, and/or expiratory time of a recumbent pattern versus expiratory time of a non-recumbent pattern.

The results of the analysis may then be telemetered to a programmer, either in real-time or at a subsequent time, for viewing by a physician. Alternatively, for an external device an indicator such as a light emitting diode or a display screen may be included to provide direct feedback to the patient or to a physician examining the patient.

Although not shown in the logical operations 300 of FIG. 3, a trending operation may also be provided for the logical operations 300 so that the result of the analysis between recumbent and non-recumbent breathing patterns may be trended over time to also allow the physician to track to the progression of the patient's congestion. Furthermore, the detection of differences in breathing patterns that are indicative of congestion may be based upon difference thresholds in rate, volume, or some combination thereof. The difference thresholds may also be based upon breathing pattern differences that are known to be normal for a population so that differences beyond this threshold are considered to be an indicator of congestion. Also, the breathing pattern information, including the patterns themselves and/or any detected differences may be communicated from the medical device for review by a physician who may make the determination of whether congestion is present.

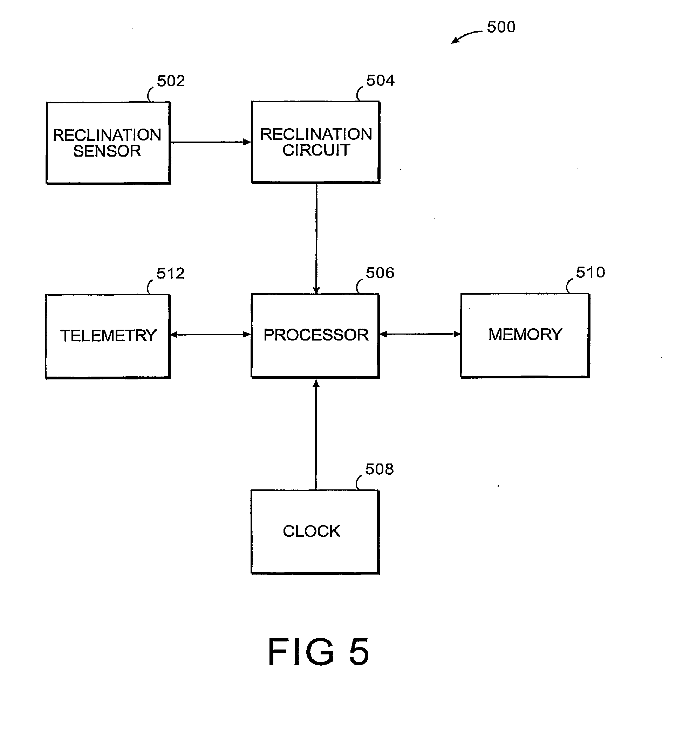

FIG. 5 shows the major components of an illustrative example of a system 500 that detects congestion by determining the amount of time a patient can remain in a recumbent position and by comparing the amount of time to a threshold related to a congestive condition. As was discussed for the system 200 of FIG. 2, any or all of the components of system 500 may be located internally or externally of the body 100. The system 500 includes a reclination sensor 502 and reclination circuit 504 as previously discussed for the system 200.

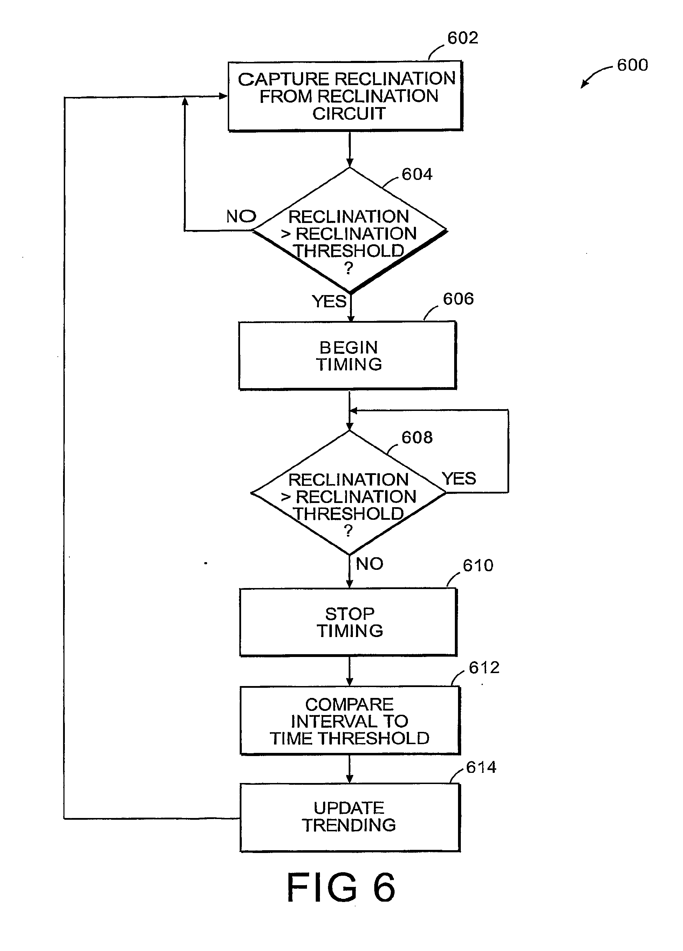

A processing device 506 such as the processing device 206 of system 200 is also included in the system 500 and receives the reclination data from the reclination circuit 504. The processor 506 utilizes the memory 510 to access programming for performing logical operations such as those discussed below with reference to FIG. 6. Additionally, the processor 506 may utilize the memory 510 to store data describing the results of the determination of the amount of time a recumbent position can be maintained and whether a congestive condition is presumed to be present. Thus, the memory 510 can be used to maintain trending information for detecting whether the patient is experiencing congestion and whether the condition is worsening over time.

Telemetry 512 may also be included as discussed above for system 200. Telemetry 512 may be used to provide an indication to a programmer as to whether the patient is presumed to be suffering from congestion. Alternatively, the telemetry may be used to provide the amount of time the patient remains in a recumbent state to the programmer for observation by a physician. Additionally, the telemetry 512 may be used to provide an update to programming for the processor 506 that is stored in memory 510.

A clock 508 is provided to allow the processor 506 to determine how long the patient remains in the recumbent position. The clock 508 may be a simple timer circuit such as a clock used to drive the operations of the processor 506 or a more elaborate time keeping device such as a clock that maintains the actual time of day. The clock 508 provides timing data or pulses to the processor 506 that the processor 506 uses to determine time intervals.

Illustrative logical operations 600 that are implemented by the processor 506 are shown in FIG. 6. The logical operations 600 allow the processor 506 to determine the amount of time the patient remains in the recumbent state. This determination then allows an inference to be drawn as to whether the patient is suffering from congestion brought on by respiratory distress upon lying down. The logical operations 600 begin at data operation 602 where the processor 506 receives reclination data from the reclination circuit 504.

Query operation 604 then detects whether the reclination is greater than a reclination threshold specified in memory 510. As discussed above, the reclination threshold may be patient specific as one patient may choose to lie down or sleep while reclined at an angle greater than another patient. If the reclination is not greater than the reclination threshold as determined at query operation 604, then the processor 506 receives the next set of reclination data at data operation 602. If the reclination is greater than the reclination threshold, then the processor 506 begins keeping track of time at timing operation 606 so that the interval from the time the patient became recumbent until the time the patient is upright can be found.

Query operation 608 detects whether the reclination is greater than the reclination threshold. If it is, then the patient is still recumbent, and query operation 608 repeats. If the reclination is no longer greater than the reclination threshold, then the processor 506 stops keeping track of time at timing operation 610. From the timing by the processor 506, an interval of time is known for the period when the patient was in the recumbent state.

In this example, the processor 506 then compares the interval of time to a time threshold that is related to a congestive condition at comparison operation 612. This threshold may be known from a study of patients suffering from congestion who experience respiratory distress after a particular amount of time in a recumbent position. Furthermore, this threshold may vary depending upon the degree of congestion that the patient is being tested for through application of the logical operations 600. Additionally, the threshold may be based upon a particular percentage of an average normal sleep time that corresponds to a particular degree of congestion, such as a patient who remains recumbent for 50% of an average normal sleep time for a population may be diagnosed as severely congested. Alternatively, the raw numbers or percentages could be trended and/or reported from the medical device to a physician who then may interpret the information to determine whether congestion is present.

For the example shown in FIG. 6, the patient's congestion level may be trended as part of an overall wellness program. Thus, upon completion of comparison operation 612, trending information stored in memory 510 or in memory of another external device may be continuously updated based on the results of the comparison operation 612 at trend operation 614. Through these trending updates, the physician can see the progression of the patient's congestion as well as additional conditions of the patient that may be simultaneously monitored with congestion.

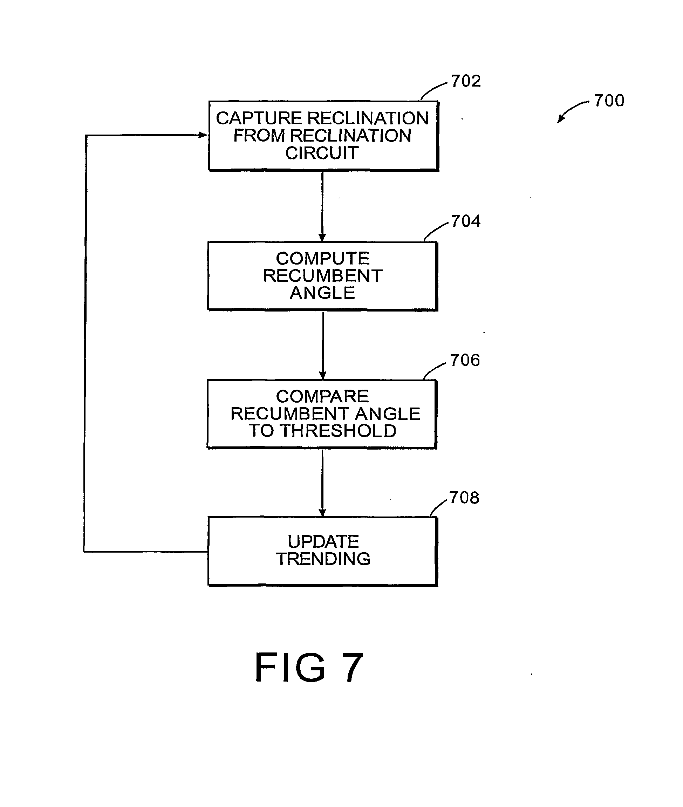

Illustrative logical operations 700 that may alternatively be implemented by the processor 506 are shown in FIG. 7 where the reclination sensor such as an accelerometer provides a signal that is representative of the recumbent angle rather than a signal that is merely indicative of a recumbent versus non-recumbent state. The logical operations 700 allow the processor 506 to obtain the recumbent angle that the patient is able to obtain for further processing and/or for transmission to a physician. The determined recumbent angle allows an inference to be drawn as to whether the patient is suffering from congestion brought on by respiratory distress upon reaching a degree of reclination.

The logical operations 700 begin at data operation 702 where the processor 506 receives reclination data from the reclination circuit 504 over a period of time. The reclination data is representative of the recumbent angles the patient obtains during the period. At process operation 704, the processor 506 computes a recumbent angle from the reclination data for the period, such as by finding the maximum recumbent angle specified by the reclination data or by averaging the recumbent angle values for the period. The period may be limited to times of day when the patient is expected or known to be resting so that there is little or no reclination data obtained while the patient is upright that would skew the average. Alternatively, a query operation may be employed by the processor 506 to throw out reclination data taken during the period that indicates an upright recumbent angle.

Comparison operation 706 then determines whether the recumbent angle is greater than a recumbent angle threshold specified in memory 510. This recumbent threshold, whose value may differ from the reclination threshold of FIG. 6, may also be patient specific as one patient may choose to lie down or sleep while reclined at a recumbent angle that is greater than another patient. If the recumbent angle is not greater than the recumbent angle threshold, then the processor 506 of this embodiment determines that the patient is suffering from congestion since it is presumed that the patient does not recline beyond the recumbent threshold due to congestion related discomfort. If the reclination is greater than the reclination threshold, then the processor 506 of this embodiment determines the patient is not suffering from congestion since it is presumed that the patient would not recline beyond the recumbent threshold due to discomfort if congestion was present.

In FIG. 7, the recumbent angle threshold may be known from a study of patients suffering from congestion who experience congestion once the recumbent angle is beyond a certain amount from vertical. Furthermore, this recumbent angle threshold may vary depending upon the degree of congestion that the patient is being tested for through application of the logical operations 700. Additionally, the recumbent angle threshold may be based upon a particular percentage of an average normal recumbent angle that corresponds to a particular degree of congestion, such as a patient who may only rest an a recumbent angle that is 50% of an average normal recumbent angle for a population may be diagnosed as severely congested.

For the example shown in FIG. 7, the patient's congestion level may be trended as part of an overall wellness program. Thus, upon completion of comparison operation 706, trending information stored in memory 510 or in memory of another external device may be continuously updated based on the results of the computation operation 704 and/or comparison operation 706 at trend operation 708. Through these trending updates, the physician can see the progression of the patient's congestion as well as additional conditions of the patient that may be simultaneously monitored with congestion.

In another example, the processor 506 may not determine whether congestion is present. Instead, the medical device may transmit the recumbent angle information to the physician who can then interpret the information to make the determination. As discussed above, the raw recumbent angle data could be trended and/or reported from the medical device to a physician who then may interpret the information to determine whether congestion is present.

While the invention has been particularly shown and described with reference to preferred embodiments thereof, it will be understood by those skilled in the art that various other changes in the form and details may be made therein without departing from the scope of the appended claims.

## Claims

1. A system for obtaining information related to congestion of a patient, comprising;
a reclination sensor (202) configured to produce a reclination signal indicative of the reclination of the patient;
detection circuitry (204) configured to produce reclination data from the reclination signal;
a respiration sensor (208) configured to produce a respiration signal indicative of respiration patterns of the patient;
respiration circuitry (210) configured to produce respiration pattern data from the respiration signal; and
a processing device (206) configured to:
monitor the respiration pattern data and the reclination data,
determine whether the patient is in a recumbent position by comparing the reclination data to data representative of a reclination threshold,
associate the respiration pattern data obtained at a point in time with the reclination data obtained at the point in time,
**characterised in that**:
the processing device (206) is configured to:
compare the respiration pattern data obtained when the patient is in a recumbent position to the respiration pattern data obtained when the patient is in a non-recumbent position to detect whether a difference is present between the respiration patterns associated with recumbent and non-recumbent positions; and
in response to differences between the respiration patterns associated with recumbent and non-recumbent positions, to determine whether the patient is suffering from congestion.

2. The system of claim 1, wherein the reclination sensor is a tilt switch.

3. The system of claim 1, wherein the reclination sensor is an accelerometer.

4. The system of claim 1, wherein the respiration sensor is configured to produce a respiration signal in response to a change in trans-thoracic impedance.

5. The system of claim 1, wherein the processing device is configured to compare the respiration pattern data taken during a non-recumbent position to respiration data taken during a recumbent position to detect whether a difference in respiration rate exists.

6. The system of claim 1, wherein the processing device is configured to compare the respiration pattern data taken during a non-recumbent position to respiration data taken during a recumbent position to detect whether a difference in tidal volume of respiration exists.

7. The system of claim 1, wherein the processing device is configured to compare the respiration pattern data taken during a non-recumbent position to respiration data taken during a recumbent position to detect whether a difference in a ratio of respiration rate to tidal volume of respiration exists.

8. The system of claim 1, further comprising a communication device (214) for transmitting the association of respiratory data with reclination data to another system.

9. The system of claim 1, wherein the respiration sensor comprises a trans-thoracic impedance sensor.

10. The system of claim 1, wherein the processing device comprises a microprocessor.

## Patentansprüche

1. System zum Erhalten von auf eine Kongestion eines Patienten bezogenen Informationen, welches aufweist:
einen Reklinationssensor (202), der konfiguriert ist zum Erzeugen eines Reklinationssignals, das die Reklination des Patienten anzeigt;
eine Erfassungsschaltung (204), die konfiguriert ist zum Erzeugen von Reklinationsdaten aus dem Reklinationssignal;
einen Atmungssensor (208), der konfiguriert ist zum Erzeugen eines Atmungssignals, das Atmungsmuster des Patienten anzeigt;
eine Atmungsschaltung (210), die konfiguriert ist zum Erzeugen von Atmungsmusterdaten aus dem Atmungssignal; und
eine Verarbeitungsvorrichtung (206), die konfiguriert ist zum:
Überwachen der Atmungsmusterdaten und der Reklinationsdaten, Bestimmen, ob der Patient in einer liegenden Position ist, durch Vergleichen der Reklinationsdaten mit Daten, die für einen Reklinationsschwellenwert repräsentativ sind,
Assoziieren der zu einem Zeitpunkt erhaltenen Atmungsmusterdaten mit den zu dem Zeitpunkt erhaltenen Reklinationsdaten,
**dadurch gekennzeichnet, dass**
die Verarbeitungsvorrichtung (206) konfiguriert ist zum:
Vergleichen der Atmungsmusterdaten, die erhalten werden, wenn sich der Patient in einer liegenden Position befindet, mit den Atmungsmusterdaten, die erhalten werden, wenn der Patient sich in einer nichtliegenden Position befindet, um zu erfassen, ob eine Differenz zwischen den Atmungsmustern, die mit der liegenden und der nichtliegenden Position assoziiert sind, vorhanden ist; und
Bestimmen, als Antwort auf Differenzen zwischen den Atmungsmustern, die mit der liegenden und der nichtliegenden Position assoziiert sind, ob der Patient unter einer Kongestion leidet.

2. System nach Anspruch 1, bei dem der Reklinationssensor ein Neigungsschalter ist.

3. System nach Anspruch 1, bei dem der Reklinationssensor ein Beschleunigungsmesser ist.

4. System nach Anspruch 1, bei dem der Atmungssensor konfiguriert ist, ein Atmungssignal als Antwort auf eine Änderung der transthorakalen Impedanz zu erzeugen.

5. System nach Anspruch 1, bei dem die Verarbeitungsvorrichtung konfiguriert ist, die Atmungsmusterdaten, die während einer nichtliegenden Position aufgenommen wurden, mit Atmungsdaten, die während einer liegenden Position aufgenommen wurden, zu vergleichen, um zu erfassen, ob eine Differenz in der Atmungsrate vorliegt.

6. System nach Anspruch 1, bei dem die Verarbeitungsvorrichtung konfiguriert ist, die Atmungsmusterdaten, die während einer nichtliegenden Position aufgenommen wurden, mit Atmungsdaten, die während einer liegenden Position aufgenommen wurden, zu vergleichen, um zu erfassen, ob eine Differenz im Tidalvolumen der Atmung vorliegt.

7. System nach Anspruch 1, bei dem die Verarbeitungsvorrichtung konfiguriert ist, die Atmungsmusterdaten, die während einer nichtliegenden Position aufgenommen wurden, mit Atmungsdaten, die während einer liegenden Position aufgenommen wurden, zu vergleichen, um zu erfassen, ob eine Differenz in einem Verhältnis der Atmungsrate zu dem Tidalvolumen der Atmung vorliegt.

8. System nach Anspruch 1, weiterhin aufweisend eine Kommunikationsvorrichtung (214) zum Übertragen der Assoziation von Atmungsdaten mit Reklinationsdaten zu einem anderen System.

9. System nach Anspruch 1, bei dem der Atmungssensor einen Sensor für transthorakale Impedanz aufweist.

10. System nach Anspruch 1, bei dem die Verarbeitungsvorrichtung einen Mikroprozessor aufweist.

## Revendications

1. Système pour obtenir une information concernant une congestion d'un patient, comprenant :
un capteur de réclinaison (202) configuré pour produire un signal de réclinaison indicatif de la réclinaison du patient ;
un circuit de détection (204) configuré pour produire des données de réclinaison à partir du signal de réclinaison ;
un capteur de respiration (208) configuré pour produire un signal de respiration indicatif de motifs de respiration du patient ;
un circuit de respiration (210) configuré pour produire des données de motif de respiration à partir du signal de respiration ; et
un dispositif de traitement (206) configuré pour :
surveiller les données de motif de respiration et les données de réclinaison ;
déterminer si oui ou non le patient est dans une position allongée en comparant les données de réclinaison avec des données représentatives d'un seuil de réclinaison ;
associer les données de motif de respiration obtenues en un point temporel avec les données de réclinaison obtenues au point temporel,
**caractérisé en ce que** le dispositif de traitement (206) est configuré pour :
comparer les données de motif de respiration obtenues lorsque le patient est dans la position allongée avec les données de motif de respiration obtenues lorsque le patient est dans une position non allongée afin de détecter si oui ou non une différence est présente entre les motifs de respiration associés à des positions allongée et non allongée ; et
en réponse à des différences entre les motifs de respiration associés à des positions allongée et non allongée, déterminer si oui ou non le patient est en train de souffrir d'une congestion.

2. Système selon la revendication 1, dans lequel le capteur de réclinaison est un commutateur d'inclinaison.

3. Système selon la revendication 1, dans lequel le capteur de réclinaison est un accéléromètre.

4. Système selon la revendication 1, dans lequel le capteur de respiration est configuré pour produire un signal de respiration en réponse à une variation de l'impédance transthoracique.

5. Système selon la revendication 1, dans lequel le dispositif de traitement est configuré pour comparer les données de motif de respiration obtenues pendant une position non allongée avec des données de respiration obtenues pendant une position allongée afin de détecter si oui ou non une différence en terme de rythme de respiration existe.

6. Système selon la revendication 1, dans lequel le dispositif de traitement est configuré pour comparer les données de motif de respiration obtenues pendant une position non allongée avec des données de respiration obtenues pendant une position allongée afin de détecter si oui ou non une différence en terme de volume tidal de respiration existe.

7. Système selon la revendication 1, dans lequel le dispositif de traitement est configuré pour comparer les données de motif de respiration obtenues pendant une position non allongée avec des données de respiration obtenues pendant une position allongée afin de détecter si oui ou non une différence en terme de rapport rythme de respiration sur volume tidal de respiration existe.

8. Système selon la revendication 1, comprenant en outre un dispositif de communication (214) pour transmettre l'association de données respiratoires avec des données de réclinaison à un autre système.

9. Système selon la revendication 1, dans lequel le capteur de respiration comprend un capteur d'impédance transthoracique.

10. Système selon la revendication 1, dans lequel le dispositif de traitement comprend un microprocesseur.
